(19) Europäisches Patentamt
European Patent Office
Office européen des brevets

(11) **EP 3 042 652 B1**

(12) **EUROPEAN PATENT SPECIFICATION**

(45) Date of publication and mention of the grant of the patent:
**29.08.2018 Bulletin 2018/35**

(21) Application number: **13893164.7**

(22) Date of filing: **05.09.2013**

(51) Int Cl.:
*A61K 31/295* (2006.01)   *A61K 31/198* (2006.01)
*A61P 35/00* (2006.01)

(86) International application number:
**PCT/CN2013/001043**

(87) International publication number:
**WO 2015/032011 (12.03.2015 Gazette 2015/10)**

(54) **USE OF COMPOSITION CONTAINING FERROUS AMINO ACID CHELATE IN PREPARATION OF ANTI-CANCER MEDICAMENT**

VERWENDUNG EINER ZUSAMMENSETZUNG MIT EISENAMINOSÄURECHELAT BEI DER HERSTELLUNG EINES MEDIKAMENTS GEGEN KREBS

UTILISATION DE COMPOSITION CONTENANT UN CHÉLATE D'ACIDE AMINÉ FERREUX POUR LA PRÉPARATION DE MÉDICAMENT ANTI-CANCÉREUX

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**13.07.2016 Bulletin 2016/28**

(73) Proprietor: **Profeat Biotechnology Co. Ltd.**
**Taoyuan City 33045 (TW)**

(72) Inventors:
• **LIN, Tsun-Yuan**
  **Taoyuan City 33045 (TW)**
• **CHEN, Mu-Kuei**
  **Taoyuan City 33045 (TW)**
• **CHEN, Tsang-Tse**
  **Taoyuan City 33045 (TW)**
• **FU, Chai-Hui**
  **Taoyuan City 33045 (TW)**
• **JAN, Hsun-Jin**
  **Taoyuan City 33045 (TW)**

• **CHIU, Wen-Cheng**
  **Taoyuan City 33045 (TW)**

(74) Representative: **Becker Kurig Straus Patentanwälte**
**Bavariastrasse 7**
**80336 München (DE)**

(56) References cited:
**EP-A- 1 658 844       EP-A1- 2 306 187**
**WO-A1-96/34602       WO-A2-03/042404**
**WO-A2-2008/039421    CN-A- 101 102 762**
**FR-M- 2 041**

• **PAOLA FERRARI ET AL: "Treatment of mild non-chemotherapy-induced iron deficiency anemia in cancer patients: Comparison between oral ferrous bisglycinate chelate and ferrous sulfate", BIOMEDICINE & PHARMACOTHERAPY, vol. 66, no. 6, 1 September 2012 (2012-09-01), pages 414-418, XP55065433, ISSN: 0753-3322, DOI: 10.1016/j.biopha.2012.06.003**

EP 3 042 652 B1

## Description

The present invention relates to a pharmaceutical composition for treating cancer, and more particularly relates to the pharmaceutical composition comprising ferrous amino acid chelate for treating cancer.

One of the main causes of death in world population is cancer or malignant tumor, wherein the mortality rates rank order is lung cancer, gastric cancer, liver cancer, colorectal cancer, breast cancer and cervical cancer. According to World Health Organization statistics (WHO), lung cancer mortality develops the fastest growing in the past two decades. Based on characteristics and clinical manifestations, lung cancers are classified into small cell lung cancer and non-small cell lung carcinoma (NSCLC). The occurrence of small cell lung cancer is higher in men, and is associated with smoking for 25%. Small cell lung cancer grows fast and spreads easily via lymph or blood to other organs. NSCLC includes squamous cell carcinoma, adenocarcinoma, and large cell carcinoma and accounts for 75% lung cancer.

Squamous cell carcinoma is also called epidermoid carcinoma, common in male smokers, and mostly extends from local to outward at early spreads via blood trail at later stage. Adenocarcinoma is the most common type of lung cancer currently, clinical symptoms of adenocarcinoma often occur after metastasis distinctly, and lung cancers suffered by non-smokers are often adenocarcinoma. Large cell carcinoma has slower growth rate, but also spreads through the blood trail and lymphoid.

Small cell lung cancer is quite sensitive to chemical and radiation therapy, but most patients will relapse within two years and generate resistance after relieving treatment. Although NSCLC grows slowly, only a quarter of the cases can receive surgical treatment at early stage, and most cases are not sensitive to chemotherapy and radiation therapy. Based on the above reasons, lung cancer patients often have poor prognosis. In addition, studies have shown that chemotherapy drugs tend to be an injury to the patient, and long-term use can cause lowered immunity, apoptosis of normal cell and lowered rate of survival. Therefore, a drug that is non-toxic to normal cells and can suppress lung cancer cell growth or apoptosis is currently in need.

In addition, liver cancer is of top ranked fatality. In addition to surgery, chemotherapy or radiation therapy often causes unbearable pain and side effects to patients. By means of inhibiting the mechanism of the progress of mutation, proliferation or spread, inhibiting hepatoma cells angiogenesis, promoting of hepatoma cells death or preventing hepatoma cell spread, target therapeutic agents are conventionally used to anti-liver cancer treatment. Moreover, since liver cancer expresses no obvious symptom at early stage, liver cancer prevention should get more attentions.

Singh et al. (Life Science, 70 49-56, 2001) disclose that after incubation with holotransferrin, which increases the concentration of ferrous iron in cancer cells, dihydroartemisinin, an analog of artemisinin, effectively killed a type of radiation-resistant human breast cancer cell in vitro. Green e, al. (Clin Cancer Res 7:3574-3579, 2001) disclose that iron chelator (2-hydroxy-1-naphthylaldehyde benzoyl hydrazone) inhibited R2 subunit of ribonucleotide reductase to inhibit the growth of breast, bladder, and head and neck cancer cell lines. EP2306187 A1 discloses ferric iron composition comprising Fe (II) or Fe (III) can be used against breast cancer.

However, Kato et al. (Int. J. Cancer: 80, 693-698, 1999) disclose increased body iron stores may increase the risk of colorectal cancer, possibly via catalyzing oxidation reactions. Simonart et al. (Gynecologic Oncology 85, 95-102, 2002) disclose Desferrioxamine and deferiprone, two chemically unrelated iron chelators, induce a time- and dose-dependent inhibition of cervical carcinoma SiHa and HeLa cell growth, block certain cell types in the G0/G1 phase, and induce the apoptosis of these cells.

In summary, the prior art does not confirm whether iron or iron compounds have cancer inhibition or therapeutic efficacy. However, chemical drugs used to treat cancer, such as lung cancer or liver cancer, often have side effects and cause patient discomfort and lead to abandonment of the treatment. Therefore, pharmaceutical products for cancer treatment that have no side effects and can be absorbed by subject are a priority.

To overcome the shortcomings of chemical drugs for treating cancer causing side effect, the objective of the present invention is to provide a pharmaceutical composition comprising a therapeutically effective amount of ferrous amino acid chelate and a pharmaceutically acceptable carrier for treating cancer.

According to the present invention, the term "ferrous amino acid chelate" as used herein refers to ferrous amino acid chelate made from mixing inorganic iron and amino acid.

Preferably, the inorganic iron includes, ferrous sulfate, ferrous chloride and ferrous pyrophosphate. The amino acid is glycine.

Preferably, the pharmaceutical composition comprises a therapeutically effective amount of ferrous amino acid chelate having 95 wt% to 100 wt % ferrous glycinate chelate. More preferably, the pharmaceutical composition comprises a therapeutically effective amount of ferrous amino acid chelate having 98 wt% to 99.9 wt % ferrous glycinate chelate.

Preferably, the therapeutically effective amount of ferrous glycine chelate is prepared from mixing inorganic iron and amino acid through 60°C to 90°C and heating for 8 hours to 48 hours to obtain the pharmaceutical composition comprising ferrous glycine chelate, wherein the weight ratio of inorganic iron and glycine is between 1: 1.2 and 1: 1.5.

According to the present invention, the pharmaceutical composition comprises the therapeutically effective amount of ferrous glycine chelate as used herein, having at least one ferrous glycine chelate, wherein the chelating ratio of ferrous and glycine of the ferrous glycine chelate is between 1: 1 and 1: 4. More preferably, the chelating ratio of ferrous and glycine of the ferrous glycine chelate is between 1: 1.5 and 1: 2.5.

Preferably, the pharmaceutical composition comprising the therapeutically effective amount of ferrous glycine chelate includes a reductant, wherein the reductant not only maintains the oxidation state of ferrous of the pharmaceutical composition comprising ferrous glycine chelate, but also enhances intestinal absorption rate of subjects. The reductant includes, ascorbic acid, citric acid, acetic acid, propionic acid, butyric acid, lactic acid, malic acid, sulfonic acid and succinic acid.

According to the present invention, the term "cancer treatment" as used herein refers to treating, relieving or inhibiting cancer. The term "therapeutically effective amount" as used herein, refers to a dosage to alleviate or inhibit progress of cancer. According to the present invention, the therapeutically effective amount for reducing, stopping, even inducing death of lung tumor or liver tumor, and the therapeutically effective amount for inhibiting lung tumor or liver tumor is determined by administering the pharmaceutical composition comprising ferrous glycine chelate in a specific amount, and measuring the tumor volume in a specific period.

According to the present invention, the term "the pharmaceutically acceptable carriers" as used herein includes any physiologically compatible and all solvents, dispersion medium, antibacterial and antifungal agents, isotonic and absorption delaying agents and analogues thereof. For example, the pharmaceutically acceptable carriers include one or more water and combination of water, salt water, phosphate buffered saline (PBS), dextrose, glycerol, ethanol and its analogues. Preferable combination includes isotonic agents, for example, sugar or polyol such as mannitol, sorbitol, or sodium chloride. The pharmaceutically acceptable carriers further include microscale auxiliary substances such as wetting or emulsifying agents, preservatives or buffers.

Preferably, the pharmaceutical composition comprising the therapeutically effective amount of ferrous glycine chelate is between 0.2 mg/kg/day and 15 mg/kg/day. More preferably, the therapeutically effective amount is between 0.3 mg/kg/day and 14 mg/kg/day. The most preferably, the therapeutically effective amount is between 0.4 mg/kg/day and 12 mg/kg/day.

In accordance with the present invention, the pharmaceutical composition for cancer treatment is prepared in multiple forms, including, liquid, semi-solid and solid dosage, such as liquid solution (including injectable and infusible solution), dispersions or suspensions, tablets, pills, powders, liposomes and suppositories. Preferred form depends on the mode of administration and therapeutic application of expectations.

Preferably, the pharmaceutical composition of the present invention is administered orally or in the form of infusion solutions, and the preferred mode of administration is enteral modes, such as orally. In an embodiment of the present invention, the pharmaceutical composition comprising ferrous glycine chelate is orally administrated.

Preferably, the pharmaceutical composition of the present invention further comprises excipient for enteral or parenteral dosage forms.

Preferably, the enteral dosage forms of the pharmaceutical composition of the present invention are oral dosage, including, solutions, suspensions, tablets and capsules.

Preferably, the cancer of the present invention includes, melanoma, liver cancer, colon cancer, lung cancer, gastric cancer, esophageal cancer, breast cancer, prostate cancer, and leukemia.

More preferably, the cancer of the present invention includes, brain tumor, low-grade astrocytoma, high-grade astrocytoma, pituitary adenoma, meningioma, CNS lymphoma, oligodendroglioma, craniopharyngioma, ependymoma, brain stem tumor, head and neck tumor, laryngeal cancer, oropharyngeal cancer, nasopharyngeal tumor, salivary gland tumor, hypopharyngeal cancer, thyroid cancer, oral cavity tumor, chest wall tumors, small cell lung cancer, non-small cell lung cancer (NSCLC), thymoma, mediastinal tumor, male breast cancer, abdomen-pelvis tumor, hepatoma, liver adenocarcinoma, gallbladder cancer, biliary tract cancer, pancreatic cancer, small intestinal tumor, large intestinal tumor, anal cancer, bladder cancer, renal cell carcinoma, cervix cancer, endometrial cancer, ovarian cancer, uterine sarcoma, and skin cancer.

More preferably, the cancer of the present invention is liver cancer or lung cancer.

By means of amino acids of small molecular weight chelating to ferrous and maintaining chelating state through the stomach, the advantage of the pharmaceutical composition in accordance with the present invention is that such pharmaceutical composition can be easily absorbed for subject and would not cause any weight variation as demonstrated by the present invention. Further, the pharmaceutical composition comprising ferrous glycine chelate obtained from preparation example 1 has better ability for inhibiting or relieving lung or liver tumors compared to the commercial ferrous amino acid (such as Ferrochel®) and inorganic iron (such as ferrous sulfate). Therefore, the pharmaceutical composition comprising ferrous glycine chelates of the present invention can be used for inhibiting or relieving cancer, particularly lung or liver cancer.

## IN THE DRAWINGS:

Fig. 1 shows the curve of tumor sizes related to nude mice (BALB/c nu/nu mice) administered with pretreatment of the pharmaceutical composition of various dosages (0.4 mg/kg/day, 1.2 mg/kg/day or 4 mg/kg/day) for 1 week, and then injected with lung cancer cell line.

Fig. 2 shows the bar chart related to weight change of nude mice administrated with the pharmaceutical composition comprising ferrous amino acid chelate of the present invention for 6 weeks.

Fig. 3 shows the tumor size fold change curve related to severe combined immunodeficient mice (SCID mice) administered with pretreatment of the pharmaceutical combination for 1 week, and then injected with liver cancer cell line.

Fig. 4 shows the survival rate curves of SCID mice injected with tumor cells within 5 weeks.

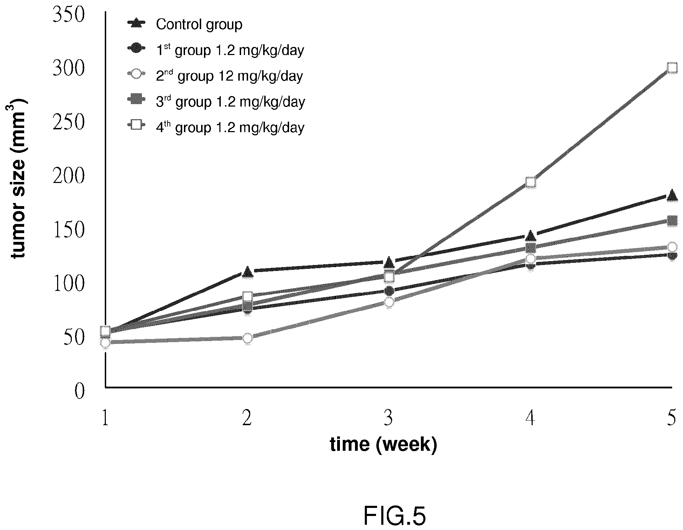

Fig. 5 illustrates that nude mice were divided into five groups, in which the control group was administered with phosphate buffer solution, the first group was administered with 1.2 mg/kg/day pharmaceutical combination comprising ferrous amino acid chelate, the second group was administered with 12 mg/kg/day pharmaceutical combination comprising ferrous amino acid chelate, the third group was administered with 1.2 mg/kg/day ferrous amino acid (Ferrochel®), and the fourth group was administered with 1.2 mg/kg/day ferrous sulfate. The nude mice in each group were administered for 7 days, and then lung cancer cells were injected into each mouse.

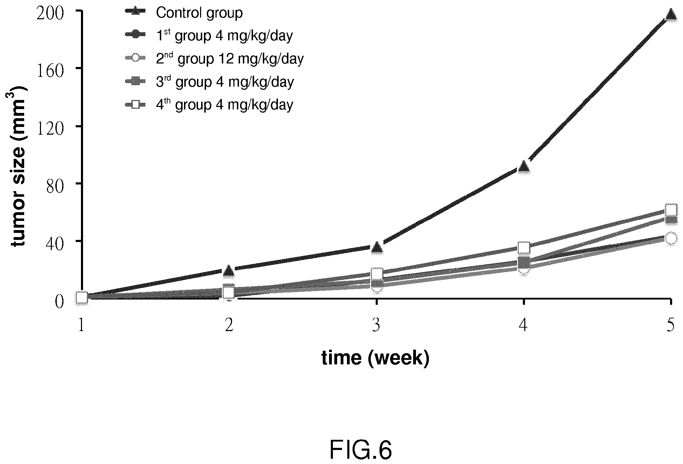

Fig. 6 illustrates that nude mice were divided into five groups, in which the control group was administered with phosphate buffer solution, the first group was administered with 4 mg/kg/day pharmaceutical combination comprising ferrous amino acid chelate of the present invention, the second group was administered with 12 mg/kg/day pharmaceutical combination comprising ferrous amino acid chelate, the third group was administered with 4 mg/kg/day ferrous amino acid (Ferrochel®), and the fourth group was administered with 4 mg/kg/day ferrous sulfate. The nude mice in each group were administered for 7 days, and then liver cancer cells were injected into each mouse.

Other objectives, advantages and novel features of the invention will become more apparent from the following detailed description when taken in conjunction with the accompanying drawings.

The pharmaceutical composition of the present invention comprising ferrous glycine chelate was used for cancer treatment, and the pharmaceutical composition comprising ferrous glycine chelate was mixed with a pharmaceutically acceptable carrier as pharmaceuticals for inhibiting or treating cancer. The pharmaceutical composition comprising ferrous glycine chelate was made from inorganic iron mixing amino acid and went through heating.

In a preferred embodiment of the present invention, the pharmaceutical composition comprising ferrous glycine chelate further included a reductant, wherein the reductant was ascorbic acid, citric acid, acetic acid, propionic acid, butyric acid, lactic acid, malic acid, sulfonic acid or succinic acid.

In a preferred embodiment of the present invention, the inorganic iron was ferrous sulfate, ferrous chloride or ferrous pyrophosphate, and the amino acid was glycine to form a pharmaceutical composition comprising ferrous amino acid chelate; the pharmaceutical composition comprising ferrous glycine chelate was proved for inhibiting cancer cell.

**Preparation example 1: Preparation of the composition comprising ferrous amino acid chelate**

The pharmaceutical composition comprising ferrous amino acid chelate was prepared as follows: firstly, ferrous sulfate and glycine (more than 98% purity) were mixed in a weight ratio of 1: 1.3 through 60°C to 90°C and heated for 8 hours to 48 hours to obtain the composition comprising ferrous amino acid chelate, wherein the chelating ratio of ferrous and amino acid of the ferrous amino acid chelate was between 1: 1 and 1: 4, and the concentration of the pharmaceutical composition comprising ferrous amino acid chelate was modulated to 0.1 $\mu$g/$\mu$l, 0.3 $\mu$g/$\mu$l, 1 $\mu$g/$\mu$l or 3 $\mu$g/$\mu$l.

**Preparation example 2: Culture of lung cancer cell**

A549 cells were seeded in DMEM medium containing 10% fetal bovine (FBS), 1% penicillin (100U/mL)-streptomycin (100 g/ml), 1% glutamine (200 mM) under 37 °C and 5% $CO_2$ about 70% to 80% cell adhesion for subculture.

**Preparation example 3: Culture of lung cancer cell**

SK Hep1 cells were seeded in DMEM medium containing 10% fetal bovine (FBS), 1% non-essential amino acid (NEAA), 1% penicillin (100U/mL)-streptomycin (100 g/ml) under 37 °C and 5% $CO_2$ for 3-4 days subculture once.

**Example 1: Volume measurement of tumor cells**

0.1 $\mu$g/$\mu$l, 0.3 $\mu$g/$\mu$l or 1$\mu$g/$\mu$l pharmaceutical composition comprising ferrous amino acid chelate obtained from preparation example 1 were respectively fed to BALB/c nu/nu mice aged 5-6 weeks (purchased from National Laboratory Animal Center, Taiwan) under 0.4 mg/kg/day, 1.2 mg/kg/day or 4 mg/kg/day and sterile water was used as a control group through 7 days. $1 \times 10^7$ cell/ml (100 $\mu$l) A549 cells obtained from preparation example 2 were respectively injected to subcutaneous of brachial artery near the hind legs of each nude mouse. After then, tumors size and volume were measured every 7 days with venier caliper, and each nude mouse was sacrificed after 6 weeks.

The tumor volume was calculated as follows:

$$(a \times b^2)/2 \ (mm^3),$$

wherein "a" was the longest diameter of a tumor, "b" was the shortest diameter.

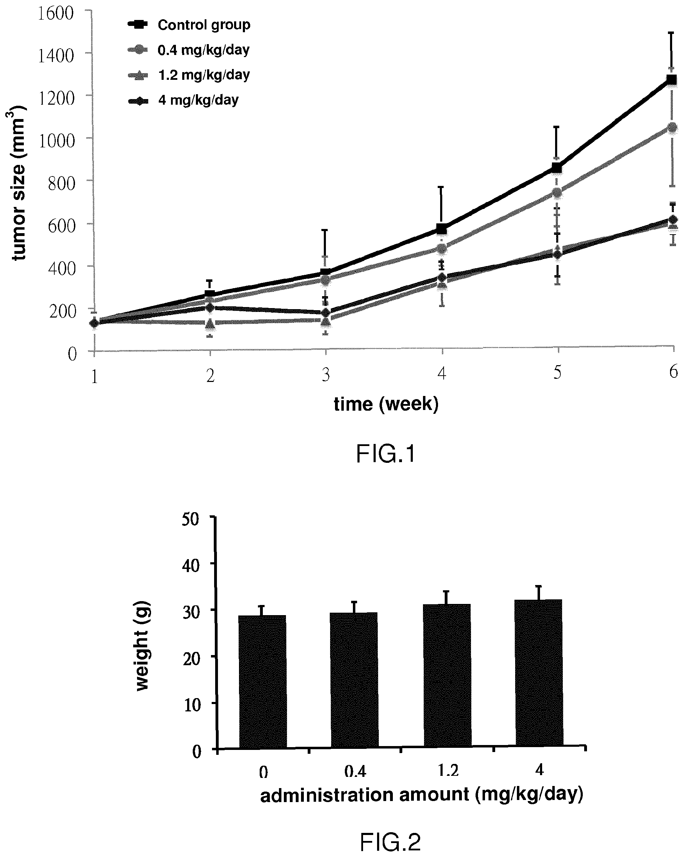

As shown in Fig.1, the volumes of lung tumors were increasing in coordination with the increasing days, when the dosage of the pharmaceutical composition comprising ferrous amino acid chelate was 1.2 mg/kg/day or 4 mg/kg/day , the dosage could inhibit tumor volume effectively. As shown in Fig.2, the growth of the nude mice would not be inhibited via the pharmaceutical composition comprising ferrous amino acid chelate.

4 mg/kg/day pharmaceutical compositions comprising ferrous amino acid chelate obtained from preparation example 1 were respectively fed to SCID mice aged about 6 weeks (purchased from Laboratory Animal Center of National Taiwan University College of Medicine) 5 times a week and sterile water was used as a control group. After 1 week, the SCID mice were anesthetized and exposed to 0.75 Gy radiations. Then, $1 \times 10^7$ cell/ml (100 $\mu$l) SK Hep1 cells obtained from preparation example 3 were respectively injected to subcutaneous of near lower back of each SCID mouse. After then, tumors size and volume were measured every 7 days with venier caliper, and each nude mouse was sacrificed after 4 weeks.

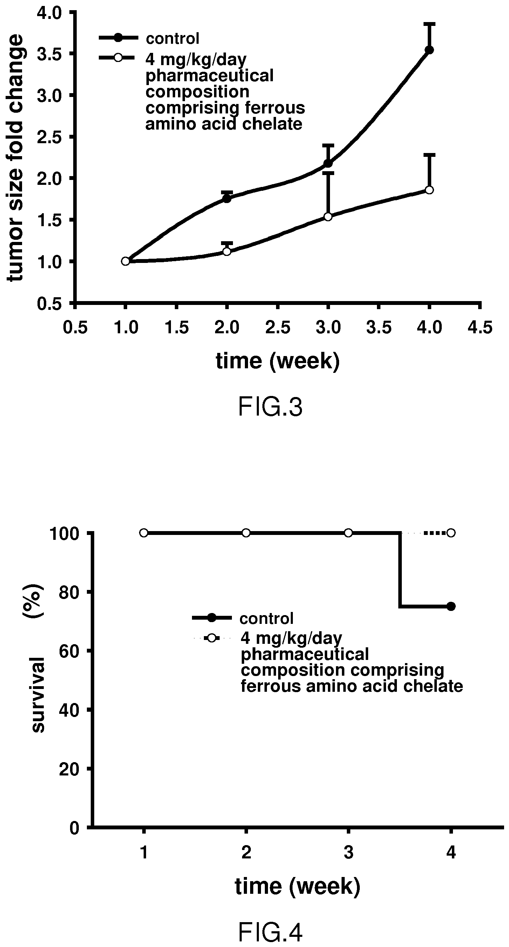

As shown in Fig.3, the volumes of liver tumors were increasing in coordination with the increasing days, and the volumes of liver tumors of the control group were 3.5 times than original tumor size at the fourth week. However, the volumes of liver tumors were only 1.8 times than original tumor size by administering 4 mg/kg/day pharmaceutical composition comprising ferrous amino acid chelate. Accordingly, the pharmaceutical composition comprising ferrous glycinate chelate was able to inhibit liver tumor volume.

**Example 2: Analysis of subject survival rate**

As shown in Fig.4, the survival rate of the control group was 75% through 4 weeks. Nevertheless, the survival rate of the SCID mice was 75% by administering 4 mg/kg/day pharmaceutical composition comprising ferrous amino acid chelate for 4 weeks. Thus, administering the pharmaceutical composition comprising ferrous amino acid chelate could enhance the survival rate of the SCID mice.

**Example 3: Control experiment of other compounds containing iron**

The nude mice were divided into 5 groups, the control group was administered with phosphate buffered saline (PBS), the first group was administered with 1.2 mg/kg/day pharmaceutical composition comprising ferrous amino acid chelate, the second group was administered with 12 mg/kg/day pharmaceutical composition comprising ferrous amino acid chelate, the third group was administered with 1.2 mg/kg/day Ferrochel® (purchased from Albion Co. Ltd.), and the fourth group was administered with 1.2 mg/kg/day ferrous sulfate (inorganic iron) for 7 days. Then, $1 \times 10^7$ cell/ml (100 $\mu$l) A549 cells obtained from preparation example 2 were respectively injected to subcutaneous of brachial artery near the hind legs of each nude mouse. After then, tumors size and volume were measured every 7 days with venier caliper, and each nude mouse was sacrificed after 6 weeks.

As shown in Fig.5, the volumes of liver tumors were increasing in coordination with the increasing days, but the volumes of liver tumors of the first and the second groups administered with 12 mg/kg/day or 1.2 mg/kg/day pharmaceutical composition comprising ferrous amino acid chelate were better inhibited than the third group administered with Ferrochel® and the fourth group administered with ferrous sulfate.

The nude mice were divided into 5 groups, the control group was administered with phosphate buffered saline (PBS), the first group was administered with 4 mg/kg/day pharmaceutical composition comprising ferrous amino acid chelate, the second group was administered with 12 mg/kg/day pharmaceutical composition comprising ferrous amino acid chelate, the third group was administered with 4 mg/kg/day Ferrochel®, and the fourth group was administered with 4 mg/kg/day ferrous sulfate (inorganic iron) for 2 weeks. Then, $1 \times 10^7$ cell/ml (100 $\mu$l) SK Hep1 cells obtained from preparation example 3 were respectively injected to subcutaneous of near lower back of each SCID mouse. After then, tumors size and volume were measured every 7 days with venier caliper, and each nude mouse was sacrificed after 5 weeks.

As shown in Fig.6, the volumes of liver tumors of the first and the second groups administered with 4 mg/kg/day or 12 mg/kg/day pharmaceutical composition comprising ferrous amino acid chelate were better inhibited than the third group administered with Ferrochel® and the fourth group administered with ferrous sulfate.

**Claims**

1. A pharmaceutical composition for use in treating cancer, **characterized in that** the pharmaceutical composition consists of a therapeutically effective amount of ferrous glycine chelate and a pharmaceutically acceptable carrier.

2. The pharmaceutical composition for use according to claim 1, **characterized in that** the chelating ratio of ferrous and glycine of the ferrous glycine chelate is between 1: 1 and 1: 4.

3. The pharmaceutical composition for use according to claim 1, **characterized in that** the chelating ratio of ferrous and glycine of the ferrous glycine chelate is between 1: 1.5 and 1: 2.5.

4. The pharmaceutical composition for use according to claim 1, **characterized in that** the therapeutically effective amount of ferrous glycine chelate is between 0.2 mg/kg/day and 15 mg/kg/day.

5. The pharmaceutical composition for use according to claim 1, **characterized in that** the therapeutically effective amount of ferrous glycine chelate is between 0.4 mg/kg/day and 12 mg/kg/day.

6. The pharmaceutical composition for use according to claim 1, **characterized in that** the therapeutically effective amount of ferrous glycine chelate is prepared from mixing inorganic iron and amino acid through 60°C to 90°C and heating for 8 hours to 48 hours to obtain the composition comprising ferrous glycine chelate, wherein the ratio of inorganic iron and glycine is between 1: 1.2 and 1: 1.5.

7. The pharmaceutical composition for use according to claim 6, **characterized in that** the inorganic iron is ferrous sulfate, ferrous chloride or ferrous pyrophosphate.

8. The pharmaceutical composition for use according to claim 1, **characterized in that** the pharmaceutical composition is in enteral or parenteral dosage form.

9. The pharmaceutical composition for use according to claim 8, **characterized in that** the enteral dosage form is oral dosage, wherein the oral dosage is solutions, suspensions, tablets or capsules.

10. The pharmaceutical composition for use according to claim 1, **characterized in that** the cancer is selected from the group consisting of melanoma, liver cancer, colon cancer, lung cancer, gastric cancer, esophageal cancer, breast cancer, prostate cancer, and leukemia.

11. The pharmaceutical composition for use according to claim 1, **characterized in that** the cancer is selected from the group consisting of brain tumor, low-grade astrocytoma, high-grade astrocytoma, pituitary adenoma, meningioma, CNS lymphoma, oligodendroglioma, craniopharyngioma, ependymoma, brain stem tumor, head and neck tumor, laryngeal cancer, oropharyngeal cancer, nasopharyngeal tumor, salivary gland tumor, hypopharyngeal cancer, thyroid cancer, oral cavity tumor, chest wall tumors, small cell lung cancer, non-small cell lung cancer (NSCLC), thymoma, mediastinal tumor, male breast cancer, abdomen-pelvis tumor, hepatoma, liver adenocarcinoma, gallbladder cancer, biliary tract cancer, pancreatic cancer, small intestinal tumor, large intestinal tumor, anal cancer, bladder cancer, renal cell carcinoma, cervical cancer, endometrial cancer, ovarian cancer, uterine sarcoma, and skin cancer.

12. The pharmaceutical composition for use according to claim 1, **characterized in that** the cancer is liver cancer or lung cancer.

**Patentansprüche**

1. Pharmazeutische Zusammensetzung zur Verwendung bei der Behandlung von Krebs, **dadurch gekennzeichnet, dass** die pharmazeutische Zusammensetzung aus einer therapeutisch wirksamen Menge von Eisen-Glycin-Chelat und einem pharmazeutisch verträglichen Träger besteht.

2. Pharmazeutische Zusammensetzung zur Verwendung nach Anspruch 1, **dadurch gekennzeichnet, dass** das Chelatisierungsverhältnis von Eisen und Glycin des Eisen-Glycin-Chelats zwischen 1 : 1 und 1 : 4 liegt.

3. Pharmazeutische Zusammensetzung zur Verwendung nach Anspruch 1, **dadurch gekennzeichnet, dass** das Chelatisierungsverhältnis von Eisen und Glycin des Eisen-Glycin-Chelats zwischen 1 : 1,5 und 1 : 2,5 liegt.

4. Pharmazeutische Zusammensetzung zur Verwendung nach Anspruch 1, **dadurch gekennzeichnet, dass** die therapeutisch wirksame Menge an Eisen-Glycin-Chelat zwischen 0,2 mg/kg/Tag und 15 mg/kg/Tag liegt.

5. Pharmazeutische Zusammensetzung zur Verwendung nach Anspruch 1, **dadurch gekennzeichnet, dass** die therapeutisch wirksame Menge an Eisen-Glycin-Chelat zwischen 0,4 mg/kg/Tag und 12 mg/kg/Tag liegt.

6. Pharmazeutische Zusammensetzung zur Verwendung nach Anspruch 1, **dadurch gekennzeichnet, dass** die therapeutisch wirksame Menge an Eisen-Glycin-Chelat durch Mischen von anorganischem Eisen und Aminosäure von 60°C bis 90°C und Erhitzen für 8 Stunden bis 48 Stunden hergestellt wird, um die das Eisen-Glycin-Chelat umfassende Zusammensetzung zu erhalten, wobei das Verhältnis von anorganischem Eisen und Glycin zwischen 1 : 1,2 und 1 : 1,5 liegt.

7. Pharmazeutische Zusammensetzung zur Verwendung nach Anspruch 6, **dadurch gekennzeichnet, dass** das anorganische Eisen Eisensulfat, Eisenchlorid oder Eisenpyrophosphat ist.

8. Pharmazeutische Zusammensetzung zur Verwendung nach Anspruch 1, **dadurch gekennzeichnet, dass** die pharmazeutische Zusammensetzung in enteraler oder parenteraler Dosierungsform vorliegt.

9. Pharmazeutische Zusammensetzung zur Verwendung nach Anspruch 8, **dadurch gekennzeichnet, dass** die enterale Darreichungsform eine orale Darreichung ist, wobei die orale Darreichung Lösungen, Suspensionen, Tabletten oder Kapseln ist.

10. Pharmazeutische Zusammensetzung zur Verwendung nach Anspruch 1, **dadurch gekennzeichnet, dass** der Krebs ausgewählt ist aus der Gruppe, bestehend aus Melanom, Leberkrebs, Darmkrebs, Lungenkrebs, Magenkrebs, Speiseröhrenkrebs, Brustkrebs, Prostatakrebs und Leukämie.

11. Pharmazeutische Zusammensetzung zur Verwendung nach Anspruch 1, **dadurch gekennzeichnet, dass** der Krebs ausgewählt ist aus der Gruppe, bestehend aus Hirntumor, niedriggradigem Astrozytom, hochgradigem Astrozytom, Hypophysenadenom, Meningiom, ZNS-Lymphom, Oligodendrogliom, Kraniopharyngeom, Ependymom , Hirnstammtumor, Kopf- und Halstumor, Kehlkopfkrebs, Oropharynxkrebs, Nasopharynxtumor, Speicheldrüsentumor, Hypopharynxkrebs, Schilddrüsenkrebs, Mundhöhlentumor, Brustwandtumoren, kleinzelligem Lungenkrebs, nicht-kleinzelligem Lungenkrebs (NSCLC), Thymom, Mediastinaltumor, männlicher Brustkrebs, Abdomen-Becken-Tumor, Hepatom, Leberadenokarzinom, Gallenblasenkrebs, Gallengangskrebs, Pankreaskrebs, Dünndarmtumor, Dickdarmtumor, Analkrebs, Blasenkrebs, Nierenzellkarzinom, Gebärmutterhalskrebs, Endometriumkrebs, Ovarialkrebs, Uterussarkom und Hautkrebs.

12. Pharmazeutische Zusammensetzung zur Verwendung nach Anspruch 1, **dadurch gekennzeichnet, dass** der Krebs Leberkrebs oder Lungenkrebs ist.

**Revendications**

1. Composition pharmaceutique pour son utilisation dans traitement du cancer, **caractérisée en ce que** la composition pharmaceutique est constituée d'une quantité thérapeutiquement efficace de chélate de glycine ferreux et d'un porteur pharmaceutiquement acceptable.

2. Composition pharmaceutique pour son utilisation selon la revendication 1, **caractérisée en ce que** le taux de chélation du ferreux et de la glycine du chélate de glycine ferreux est compris entre 1/1 et 1/4.

3. Composition pharmaceutique pour son utilisation selon la revendication 1, **caractérisée en ce que** le taux de chélation du ferreux et de la glycine du chélate de glycine ferreux est compris entre 1/1,5 et 1/2,5.

4. Composition pharmaceutique pour son utilisation selon la revendication 1, **caractérisée en ce que** la quantité thérapeutiquement efficace de chélate de glycine ferreux est comprise entre 0,2 mg/kg/jour et 15 mg/kg/jour.

5. Composition pharmaceutique pour son utilisation selon la revendication 1, **caractérisée en ce que** la quantité thérapeutiquement efficace de chélate de glycine ferreux est comprise entre 0,4 mg/kg/jour et 12 mg/kg/jour.

6. Composition pharmaceutique pour son utilisation selon la revendication 1, **caractérisée en ce que** la quantité thérapeutiquement efficace de chélate de glycine ferreux est préparée par mélange de fer inorganique et d'acide aminé à 60 °C à 90 °C et chauffage pendant 8 heures à 48 heures pour obtenir la composition comprenant le chélate de glycine ferreux, dans laquelle le rapport de fer inorganique et de la glycine est compris entre 1/1,2 et 1/1,5.

7. Composition pharmaceutique pour son utilisation selon la revendication 6, **caractérisée en ce que** le fer inorganique est le sulfate ferreux, le chlorure ferreux ou le pyrophosphate ferreux.

8. Composition pharmaceutique pour son utilisation selon la revendication 1, **caractérisée en ce que** la composition pharmaceutique se trouve sous une forme galénique entérale ou parentérale.

9. Composition pharmaceutique pour son utilisation selon la revendication 8, **caractérisée en ce que** la forme galénique entérale est un dosage oral, dans lequel le dosage oral est des solutions, des suspensions, des comprimés ou des capsules.

10. Composition pharmaceutique pour son utilisation selon la revendication 1, **caractérisée en ce que** le cancer est sélectionné dans le groupe constitué du mélanome, du cancer du foie, du cancer du côlon, du cancer du poumon, du cancer gastrique, du cancer de l'oesophage, du cancer du sein, du cancer de la prostate, et de la leucémie.

11. Composition pharmaceutique pour son utilisation selon la revendication 1, **caractérisée en ce que** le cancer est sélectionné dans le groupe constitué d'une tumeur du cerveau, d'un astrocytome de bas grade, d'un astrocytome de haut grade, d'un adénome hypophysaire, d'un méningiome, d'un lymphome du SNC, d'un oligodendrogliome, d'un craniopharyngiome, d'un épendymome, d'une tumeur du tronc cérébral, d'une tumeur de la tête et du cou, d'un cancer du larynx, d'un cancer de l'oropharynx, d'une tumeur du nasopharynx, d'une tumeur de la glande salivaire, d'un cancer de l'hypopharynx, du cancer de la thyroïde, d'une tumeur de la cavité buccale, de tumeurs de la paroi thoracique, du cancer du poumon à petites cellules, du cancer du poumon non à petites cellules (NSCLC), d'un thymome, d'une tumeur médiastinale, du cancer du sein chez l'homme, d'une tumeur abdomino-pelvienne, d'un hépatome, d'un adénocarcinome hépatique, du cancer de la vésicule biliaire, du cancer du canal biliaire, du cancer du pancréas, d'une tumeur de l'intestin grêle, d'une tumeur du côlon, du cancer anal, du cancer de la vessie, d'un carcinome des cellules rénales, du cancer du col de l'utérus, du cancer de l'endomètre, du cancer de l'ovaire, d'un sarcome de l'utérus, et du cancer de la peau.

**12.** Composition pharmaceutique pour son utilisation selon la revendication 1, **caractérisée en ce que** le cancer est le cancer du foie ou le cancer du poumon.

FIG.1

FIG.2

FIG.3

FIG.4

FIG.5

FIG.6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2306187 A1

**Non-patent literature cited in the description**

- **SINGH et al.** *Life Science,* 2001, vol. 70, 49-56
- **GREEN.** *Clin Cancer Res,* 2001, vol. 7, 3574-3579
- **KATO et al.** *Int. J. Cancer,* 1999, vol. 80, 693-698
- **SIMONART et al.** *Gynecologic Oncology,* 2002, vol. 85, 95-102